# EUROPÄISCHE PATENTANMELDUNG

(11) **EP 0 756 845 A1**
(43) Veröffentlichungstag der Anmeldung: **05.02.1997**
(21) Anmeldenummer: 96109839.9
(22) Anmeldetag: 19.06.1996
(51) Int. Cl.: A61B 1/00, G02B 23/24

(54) **Endoskopoptik mit eine Lichtblende aufweisender Fensterplatte**

(30) Priorität: 17.07.1995 DE 19525995
(71) Anmelder: OLYMPUS WINTER & IBE GmbH, 22045 Hamburg (DE)
(72) Erfinder: Wulfsberg, Jens Peter, Dr.-Ing., 22949 Ammersbek (DE)
(74) Vertreter: Schaefer, Konrad, Dipl.-Phys.

(57) **Zusammenfassung**

Eine Endoskopoptik mit einem Schaft (1), in dem parallel nebeneinander ein Bildleiter (3) und ein Lichtleiter (5) angeordnet sind, deren distale Enden von einem das distale Ende des Schaftes verschließenden Fenster (6) abgedeckt sind, das im Bereich der Trennlinie (2) zwischen den Querschnittsbereichen des Bildleiters (3) und des Lichtleiters (5) eine Lichtblende (12-20) aufweist, ist dadurch gekennzeichnet, daß das Fenster (6) als einstückige Fensterplatte (6) ausgebildet ist und daß als Lichtblende (12-20) wenigstens eine in einer der parallelen Oberflächen der Fensterplatte (6) eingebrachte, parallel zur Trennlinie (2) erstreckte Nut (12-20) vorgesehen ist.

## Beschreibung

Die Erfindung betrifft eine Endoskopoptik der im Oberbegriff des Anspruches 1 genannten Art.

Solche Optiken werden zumeist als Einsätze in endoskopischen Instrumenten verwendet, überwiegend bei medizinischer Anwendung, und weisen in einem Schaft einen Bildleiter und einen Lichtleiter auf, wobei der Bildleiter zur Betrachtung dient, also mit einem distal angeordneten Objektiv Licht vom Beobachtungsort aufnimmt, während der parallel daneben angeordnete Lichtleiter Licht nach vorn auf den Beobachtungsort abstrahlt.

Die Optik kann flexibel oder starr ausgebildet sein. In letzterem Fall ist der Bildleiter üblicherweise als Stablinsenanordnung ausgebildet. Der Lichtleiter ist üblicherweise stets als Bündel lichtübertragender Fasern ausgebildet.

Problematisch ist bei solchen Endoskopoptiken der Schutz des Bildleiters und des Lichtleiters gegen von außen eindringede Medien, insbesondere Wasser. Insbesondere bei medizinischen Optiken, die mit Heißdampf sterilisiert werden, entstehen hierbei große Abdichtungsprobleme.

Am besten läßt sich das Abdichtungsproblem am distalen Ende des Schaftes durch Fenster lösen, die im Schaft diesen verschließend abgedichtet befestigt sind und die den Bildleiter und den Lichtleiter abdecken.

Dabei kommt es aber zu Problemen mit Lichtüberstrahlung aus dem Lichtleiter in den Bildleiter durch Reflexion in den Fenstern.

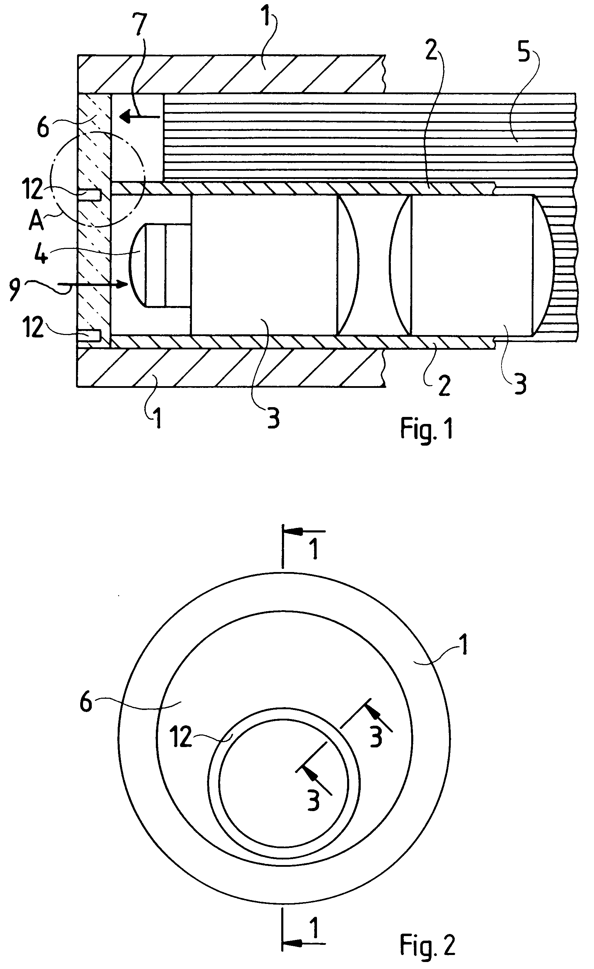

Aus der
DE 37 08 124 A1
ist eine Konstruktion bekannt (Fig. 1), bei der nur der Bildleiter mit einem Fenster abgedeckt ist. Überstrahlungsprobleme werden dadurch vermieden. Die Abdichtung des Instrumentes, insbesondere des Lichtleiters, ist dabei aber ungenügend. Fig. 16 zeigt eine Konstruktion, bei der Lichtleiter und Bildleiter mit getrennt gefaßten Fenstern abgedeckt sind. Die Fassung zwischen den Fenstern verhindert als Lichtblende die Lichtüberstrahlung vom Lichtleiter zum Bildleiter. Konstruktiv und hinsichtlich der Abdichtungsprobleme ist diese Konstruktion aber schwierig.

Aus der
DE 90 16 829 U1
ist eine Endoskopoptik bekannt mit einer einstückig durchgehend sowohl den Bildleiter als auch den Lichtleiter abdeckenden Fensterplatte. Maßnahmen zur Verhinderung der Lichtüberstrahlung sind hier aber nicht getroffen.

Aus der gattungsgemäßen
DE 42 11 547 A1
ist eine Konstruktion bekannt, bei der Lichtleiter und Bildleiter von einem gemeinsamen Fenster abgedeckt sind, das jedoch mehrstückig zusammengesetzt ist, wobei die aneinandergrenzenden Ränder der Stücke dieses Fensters mit einer Schwärzung oder Bedampfung als Lichtblende versehen sind. Lichtüberstrahlung wird hier vermieden. Es bestehen aber die erheblichen Dichtigkeits- und Festigkeitsprobleme einer zusammengesetzten Glasplatte.

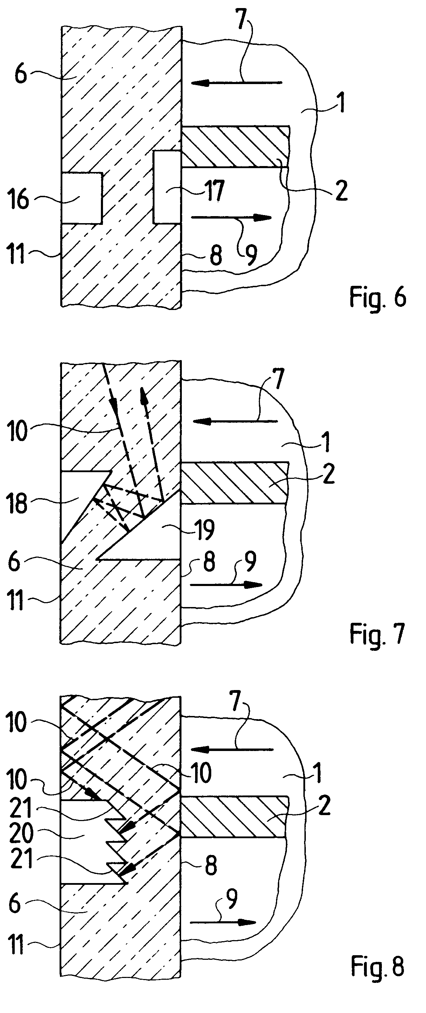

Weiterhin ist aus der
DE 39 23 007 C2
(ähnlich: JP 6-23811 B2 sowie JP 2-132409 A)
eine Konstruktion bekannt, bei der ein Fenster als einstückig den gesamten Schaft, also sowohl den Lichtleiter als auch den Bildleiter abdeckende Fensterplatte vorgesehen ist, die im Bereich der Trennlinie zwischen Bildleiter und Lichtleiter keine Lichtblende aufweist, also völlig lichtdurchlässig ist, dort aber aufgrund einer keilförmigen Gestaltung eine Lichtfalle ausbildet. Dabei kann gegebenenfalls (Fig. 7 und 8) eine der beiden Oberflächen der Platte in geeigneter Weise reflexionsmindernd ausgebildet sein. Vorteilhaft ist hierbei die stabile einstückige Ausbildung der Fensterplatte, mit der Dichtigkeitsprobleme hervorragend lösbar sind. Nachteilig bei dieser Konstruktion ist allerdings die aufwendige keilförmige Ausbildung der Fensterplatte und insbesondere die erforderliche große Breite der Lichtfalle. Bildleiter und Lichtleiter müssen daher in größerem Abstand vorgesehen sein, was zu einer unerwünschten Durchmesservergrößerung der Endoskopoptik führt.

Diese Schrift (Fig. 8) zeigt auch die Möglichkeit, eine der Oberflächen der Fensterplatte mit Nuten zu versehen. Jedoch sind diese sehr flach ausgebildet und dienen lediglich dazu, unter großflächiger Anbringung vieler Nuten nach Art einer Riffelung die Oberfläche der Lichtfalle reflexionsmindernd zu gestalten.

Aufgabe der vorliegenden Erfindung ist es, eine Endoskopoptik der gattungsgemäßen Art zu schaffen, die bei wirkungsvoller Verhinderung der Lichteinspiegelung aus dem Lichtleiter in den Bildleiter und bei kompakter Querschnittsausbildung eine dauerhafte Abdichtung des Inneren der Endoskopoptik gewährleistet.

Diese Aufgabe wird erfindungsgemäß mit den Merkmalen des Kennzeichnungsteiles des Anspruches 1 gelöst.

Erfindungsgemäß ist die Fensterplatte einstückig ausgebildet, also nur an ihrem Rand mit dem Schaft der Endoskopoptik abgedichtet verbunden, was ohne Probleme zu langdauernder guter Abdichtung des Inneren der Endoskopoptik führt. Die Lichtblende ist in Form einer oder weniger Nuten ausgebildet, die in die Oberflächen der Fensterplatte eingebracht sind und die sich wie Querwände quer zu den störenden Lichtstrahlen erstrecken, die bei Reflexion vom Lichtleiter in den Bildleiter innerhalb der Lichtplatte im wesentlichen quer zur Achse des Schaftes der Endoskopoptik verlaufen. Die Querreflexion in der Fensterplatte kann dadurch erheblich verringert werden, wobei die Nuttiefe in Abwägung zur verbleibenden mechanischen Festigkeit der Fensterplatte den Grad der Reflexionsunterdrückung bestimmt.

Vorteilhaft können mehrere Nuten, insbesondere gemäß Anspruch 2 zwei Nuten vorgesehen sein, die eine wirkungsvollere Abblendung der Lichtreflexion ermöglichen, und zwar auch bei geringerer, die mechanische Stabilität der Fensterplatte weniger beeinträchtigenderer Tiefe.

Vorteilhaft sind dabei die Merkmale des Anspruches 3 vorgesehen. Auf diese Weise läßt sich eine besonders wirksame Unterbindung der Querreflexion erreichen, wobei vorteilhaft gemäß Anspruch 4 die Nuten in einer labyrinthartigen Anordnung vorgesehen sind.

Vorteilhaft sind die Merkmale des Anspsruches 5 vorgesehen. Auf diese Weise läßt sich unter geschickter Anordnung der abgewinkelt vorgesehenen Seitenwände der Nuten das quer durch die Fensterplatte vom Lichtleiter zum Bildleiter reflektierende Licht besonders wirksam am Übertritt hindern.

Vorteilhaft sind die Merkmale des Anspruches 6 vorgesehen. Auf diese Weise kann durch Rückreflexion der Lichtstrahlen an den schrägstehenden Flächen des Nutengrundes auch dort das Licht am Übertritt in den Bereich des Bildleiters gehindert werden.

In den Zeichnungen ist die Erfindung beispielsweise und schematisch dargestellt. Es zeigen:
- Fig. 1: in einem Achsschnitt gemäß Linie 1 - 1 in Fig. 2 den distalen Endbereich einer erfindungsgemäßen Endoskopoptik in einer ersten Ausführungsform,
- Fig. 2: eine Frontansicht in Achsrichtung auf die Anordnung gemäß Fig. 1,
- Fig. 3: einen vergrößerten Schnitt durch die Fensterplatte gemäß Linie 3 - 3 in Fig. 2 im Bereich des Ausschnittes A in Fig. 1,
- Fig. 4 - 8: Schnitte gemäß Fig. 3 durch weitere Ausführungsformen der Fensterplatte mit unterschiedlichen Nutanordnungen.

Fig. 1 zeigt im Achsschnitt den distalen Endbereich einer starren Endoskopoptik, wie sie häufig für medizinische Zwecke verwendet wird. Die Optik ist umschlossen von einem als rundes Rohr ausgebildeten Schaft 1, in dessen Innerem exzentrisch ein Bildleiterrohr 2 angeordnet ist, das den Bildleiter in Form eines Linsenstapels, gebildet aus Stablinsen 3, aufnimmt. Auf der distal vordersten Stablinse 3 ist eine Objektivlinse 4 aufgekittet.

Der bei dieser üblichen Anordnung sichelförmig das Bildleiterrohr 2 umgebende Querschnittsbereich innerhalb des Schaftes 1 ist vollständig mit einem Lichtleiter 5 ausgefüllt, der aus einem Bündel paralleler Lichtleitfasern gebildet ist. Die distale Endfläche des Lichtleiters 5 ist plan geschliffen.

Die distale Öffnung des Schaftes 1 ist von einer Fensterplatte 6 verschlossen, die mit ihrem Rand durch geeignete Maßnahmen, beispielsweise Verlötung, oder auch mittels eines innen und außen verlöteten Fensterhalters an der Innenwand des Schaftes 1 flüssigkeits- und gasdicht befestigt ist.

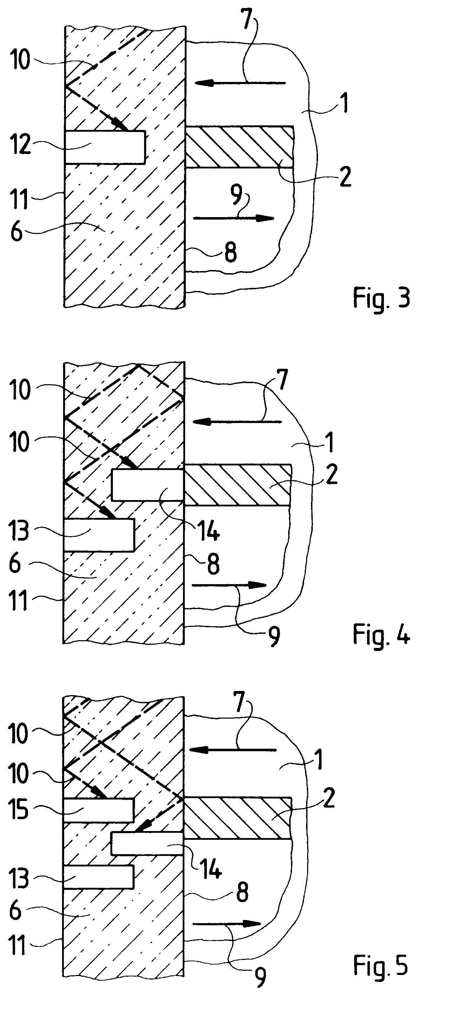

Wie Fig. 3 in einem stark schematisierten vergrößerten Schnitt durch die Fensterplatte im Bereich der Trennlinie zwischen dem Querschnittsbereich des Lichtleiters 5 und des innerhalb des Bildleiterrohres 2 liegenden Bildleiters zeigt, strahlt aus dem Lichtleiter Licht in Richtung des Pfeiles 7 auf die Innenfläche 8 der Fensterplatte 6. Im Bereich des Bildleiters durch die Fensterplatte in Richtung des Pfeiles 9 kommendes Licht wird von der Objektivlinse 4 des Bildleiters aufgefangen.

Die den Bildleiter 5 bildenden lichtleitenden Fasern strahlen an ihren Endflächen Licht aber nicht nur parallel in Richtung des Pfeiles 7, sondern auch unter Schrägwinkeln ab. Dieses Licht kann, wie der gestrichelte Pfeil 10 zeigt, an der Außenseite 11 der Fensterplatte 6 reflektiert werden und gegebenenfalls nach zick-zack-förmiger Mehrfachreflexion an Innenseite 8 und Außenseite 11 der Fensterplatte 6 über die durch das Bildleiterrohr 2 gebildete Trennwand hinweg in den Bereich des Bildleiters und auf die Objektivlinse 4 gelangen. Diese äußerst störenden Lichtreflexionen sind zu vermeiden.

Zu diesem Zweck ist in der ersten, in den Fig. 1 bis 3 dargestellten Ausführungsform eine Nut 12 vorgesehen, die im Bereich der Trennlinie zwischen den Querschnittsbereichen des Schaftes 1, die vom Bildleiter bzw. vom Lichtleiter 5 ausgefüllt sind, angeordnet ist, also die Projektion des Bildleiterrohres 2 auf die Fensterplatte 6 bildet. Die Nut 12 ist im Bereich dieser Trennlinie von der Außenseite 11 her in die Fensterplatte 6 eingebracht. Im dargestellten Ausführungsbeispiel handelt es sich um eine Rechtecknut, die sich etwa über zwei Drittel der Dicke der Fensterplatte 6 erstreckt.

Wie Fig. 3 zeigt, werden die meisten in der Fensterplatte 6 quer von dem Lichtleiter 5 zum Bildleiter über das Bildleiterrohr 2 hinweg reflektierten Lichtstrahlen 10 an der Seitenfläche der Nut 12 rückreflektiert oder auf sonstige Weise am Übertritt gehindert. Die Oberflächen der Nut 12 können zu diesem Zweck poliert, also hochreflektierend oder matt, also das Licht diffus in die Nut hinein abgebend ausgebildet sein. Auf jeden Fall gelangen nur geringe Lichtmengen nach Mehrfachreflexion unter der Nut 12 hindurch in den Bereich des Bildleiters, so daß die Reflexionsstörungen zumindest wesentlich verringert werden.

Fig. 4 zeigt im Schnitt der Fig. 3 eine Variante, bei der zwei Nuten 13 und 14 vorgesehen sind. Diese verlaufen parallel, wobei die Nut 13 von der Außenseite 11 her und die Nut 14 von der Innenseite 8 her in die Fensterplatte 6 eingebracht ist. Durch diese labyrinthartige Anordnung werden Querreflexionen im Sinne der gestrichelten Pfeile 10 besonders wirksam vermieden.

Fig. 5 zeigt eine Variante, die der der Fig. 4 entspricht, jedoch zusätzlich zu den dort vorgesehenen Nuten 13 und 14 eine weitere Nut 15 auf der Außenseite 11 der Fensterplatte 6 aufweist. Dadurch wird die Labyrinthwirkung weiter verstärkt, so daß Lichtübertritt durch die Fensterplatte 6 vom Bereich des Lichtleiters in den Bereich des Bildleiters nahezu vollständig verhindert wird.

Fig. 6 zeigt eine Variante, bei der zwei flache Nuten 16 und 17 gegenüberliegend in der Außenseite 11 und der Innenseite 8 der Fensterplatte 6 vorgesehen sind. Wie dargestellt, können die Nuten von unterschiedlicher Breite sein. Auch diese Anordnung ergibt eine wirksame Verringerung der Lichtreflexionen.

Fig. 7 zeigt eine Variante, bei der zwei gegenüberliegend angeordnete Nuten 18 und 19 im Querschnitt dreieckförmig ausgebildet sind. Die Nuten weisen je eine lotrecht zur Oberfläche der Fensterplatte 6 angeordnete und je eine schrägstehende Wand auf. Die schrägstehenden Wände sorgen unter geschickter Anordnung zur Hauptrichtung der abzufangenden Reflexionslichtstrahlen für eine wirksame labyrinthartige Unterdrückung der Lichtüberstrahlung. Dabei können vorteilhaft, wie in Fig. 7 dargestellt, die schrägstehenden Wände der beiden Nuten auch zueinander schrägstehen und zwischen sich eine Lichtfalle der in der DE 39 23 007 C2 beschriebenen Art ausbilden.

Fig. 8 zeigt eine Variante, die ähnlich wie die der Ausführungsformen der Fig. 1 bis 3 nur eine außenliegende Nut 20 aufweist, die relativ flach ausgebildet ist. Am Grund der Nut, der parallel zur Oberfläche der Fensterplatte 6 verläuft, könnten reflektierende Lichtstrahlen zum Bereich des Bildleiters reflektiert werden. Daher ist der Nutengrund streifenweise mit schrägstehenden Flächen 21 ausgebildet, die in ihrer Richtung derart angeordnet sind, daß sie aus dem Bereich des Lichtleiters schräg einfallende Lichtstrahlen zurückreflektieren.

Es sind weitere Nutanordnungen möglich, beispielsweise in Kombination der dargestellten Ausführungsformen.

## Patentansprüche

1. Endoskopoptik mit einem Schaft (1), in dem parallel nebeneinander ein Bildleiter (3) und ein Lichtleiter (5) angeordnet sind, deren distale Enden von einem das distale Ende des Schaftes (1) verschließenden Fenster (6) abgedeckt sind, das im Bereich der Trennlinie (2) zwischen den Querschnittsbereichen des Bildleiters und des Lichtleiters eine Lichtblende (12 - 20) aufweist, **dadurch gekennzeichnet**, daß das Fenster als einstückige Fensterplatte (6) ausgebildet ist und daß als Lichtblende wenigstens eine in einer der parallelen Oberflächen (8, 11) der Fensterplatte (6) eingebrachte, parallel zur Trennlinie (2) erstreckte Nut (12 - 20) vorgesehen ist.

2. Endoskopoptik nach Anspruch 1, **dadurch gekennzeichnet**, daß zwei Nuten (13, 14; 16, 17; 18, 19) vorgesehen sind.

3. Endoskopoptik nach Anspruch 2, **dadurch gekennzeichnet**, daß die Nuten (13, 14; 16, 17; 18, 19) in unterschiedlichen Oberflächen (11, 8) der Fensterplatte (6) angeordnet sind.

4. Endoskopoptik nach Anspruch 3, **dadurch gekennzeichnet**, daß die Nuten (13, 14; 13, 14, 15) seitlich versetzt angeordnet sind.

5. Endoskopoptik nach Anspruch 1, **dadurch gekennzeichnet**, daß die Nut (18, 19) wenigstens eine Seitenwand aufweist, die unter einem Winkel zur Lotrechten auf der Oberfläche (8, 11) der Fensterplatte (6) angeordnet ist.

6. Endoskopoptik nach Anspruch 1, **dadurch gekennzeichnet**, daß der Nutengrund (21) im Ganzen oder bereichsweise unter einem Winkel zur Oberfläche (11) der Fensterplatte (6) angeordnet ist.
